# EUROPEAN PATENT APPLICATION

(11) **EP 4 070 846 A1**
(43) Date of publication of application: **12.10.2022**
(21) Application number: 20896862.8
(22) Date of filing: 01.12.2020
(51) Int. Cl.: A61M 25/00, A61M 25/092

(54) **CATHETER**

(30) Priority: 02.12.2019 JP 2019217810
(71) Applicant: Asahi Intecc Co., Ltd., Seto-shi, Aichi 489-0071 (JP)
(72) Inventor: NAMIMA Satoshi, Seto-shi, Aichi 489-0071 (JP)
(74) Representative: Winter, Brandl - Partnerschaft mbB
(86) International application number: PCT/JP2020/044631
(87) International publication number: WO 2021/112067

(57) **Abstract**

To provide a catheter including a main lumen, a plurality of sub lumens arranged around the main lumen and spirally formed around the main lumen, a plurality of operating wires inserted into each of the plurality of sub lumens, a distal end flexible portion arranged on a distal end side of the catheter and curvable by the plurality of operating wires, and a holding portion arranged at a distal end of the distal end flexible portion and holding each of the plurality of operating wires, in which each of the plurality of sub lumens is wound by a half turn or more in the distal end flexible portion.

## Description

### TECHNICAL FIELD

The present invention relates to a catheter.

### BACKGROUND ART

There is known a catheter used by inserting into a living lumen such as a vascular system, a lymph gland system, a biliary system, a urinary tract system, a respiratory system, a digestive system, a secretory gland, and a reproductive organ. In such a catheter, there is disclosed a technique for curving the catheter in a direction intersecting a longitudinal direction by using an operation thread such as an operating wire to position a distal end side of the catheter in the vicinity of a living tissue (for example, see Patent Literatures 1 to 3).

### CITATION LIST

### Patent Literature

Patent Literature 1: JP 2015-188569 A
Patent Literature 2: JP 2014-111139 A
Patent Literature 3: JP 2015-159969 A

### SUMMARY OF INVENTION

### TECHNICAL PROBLEM

The techniques described in Patent Literatures 1 and 2 have a problem in operability of the operating wire or the like in a state where a distal end portion is curved. The technique described in Patent Literature 3 has a problem in that although there is provided a structure including an operation-thread sub lumen capable of moving in a circumferential direction, which is a structure that can respond to a change in natural length due to differences in vascular routes, if the distal end portion is curved greatly, it is not easy to perform a stable operation into the circumferential direction of the operation-thread sub lumen.

The present invention has been made to solve the above-mentioned problems, and an object thereof is to improve operability in a curvable catheter.

### SOLUTION TO PROBLEM

The present invention has been contrived to solve at least some of the above-described problems, and can be realized as the following aspects.
(1) According to one aspect of the present invention, a catheter is provided. The catheter includes a main lumen, a plurality of sub lumens arranged around the main lumen and spirally formed around the main lumen, a plurality of operating wires inserted into each of the plurality of sub lumens, a distal end flexible portion arranged on a distal end side of the catheter and curvable by the plurality of operating wires, and a holding portion arranged at a distal end of the distal end flexible portion and holding each of the plurality of operating wires, in which each of the plurality of sub lumens is wound by a half turn or more in the distal end flexible portion.
   According to such a configuration, each of the plurality of sub lumens is wound by a half turn or more in the distal end flexible portion. Therefore, as a result of the operating wires being operated, if the distal end flexible portion of the catheter is curved, tension is exerted on a part corresponding to the outside of the curved portion in the sub lumen, and tension is loosened in a part corresponding to the inside of the curved portion. As a result, the tensions exerted on the sub lumen are canceled out, and thus, a drag force when the operating wires are operated is reduced, and it is possible to improve the operability. The plurality of sub lumens are each formed in a spiral shape, and thus, if any operating wire, of the plurality of operating wires, is pulled, the drag force exerted on each operating wire is reduced, and it is possible to improve the operability.
(2) In the catheter of the above aspect, the plurality of sub lumens may be each arranged at substantially equal intervals in a circumferential direction of the main lumen. With such a configuration, the plurality of sub lumens are arranged at equal intervals in the circumferential direction of the main lumen, and thus, it is possible to appropriately operate the distal end flexible portion in a curving direction.
(3) The catheter of the above aspect may be provided with a hollow tubular main tube configuring the main lumen, a plurality of hollow tubular sub tubes configuring each of the plurality of sub lumens; and a hollow tubular outer tube containing the main tube and the sub tubes. With such a configuration, it is possible to provide a catheter that can be easily manufactured.
(4) In the catheter of the above aspect, the plurality of sub lumens may include a first sub lumen and a second sub lumen, and the plurality of operating wires may include a first operating wire inserted into the first sub lumen and a second operating wire inserted into the second sub lumen. With such a configuration, it is possible to provide a catheter that can be easily operated and manufactured.

The disclosed embodiments can be realized in various aspects, and for example, can be realized in a mode such as a catheter, a main body portion for the catheter, and a method of manufacturing the catheter and the main body portion.

### BRIEF DESCRIPTION OF DRAWINGS

Fig. 1 is an explanatory diagram illustrating a configuration of a catheter of a first embodiment.
Fig. 2 is an explanatory diagram illustrating a catheter with a distal end flexible portion being curved.
Fig. 3 is an explanatory diagram illustrating a longitudinal sectional configuration of a part of a distal end flexible portion of the catheter.
Fig. 4 is an explanatory diagram of a configuration of the distal end flexible portion.
Fig. 5 is a cross-sectional view taken along A-A of the distal end flexible portion in Fig. 4.
Fig. 6 is a cross-sectional view taken along B-B of the distal end flexible portion in Fig. 4.
Fig. 7 is a cross-sectional view taken along C-C of the distal end flexible portion in Fig. 4.
Fig. 8 is a cross-sectional view taken along D-D of the distal end flexible portion in Fig. 4.
Fig. 9 is an explanatory diagram of a configuration of a catheter of the prior art.
Fig. 10 is an explanatory diagram illustrating a case where a distal end flexible portion of the catheter of the prior art is curved.
Fig. 11 is an explanatory diagram of an effect of the catheter of the first embodiment.
Fig. 12 is an explanatory diagram of a configuration of a distal end flexible portion of a catheter of a second embodiment.
Fig. 13 is a cross-sectional view taken along B-B of the distal end flexible portion in Fig. 12.
Fig. 14 is a cross-sectional view taken along C-C of the distal end flexible portion in Fig. 12.
Fig. 15 is a cross-sectional view taken along D-D of the distal end flexible portion in Fig. 12.

### EMBODIMENTS OF THE INVENTION

### <First Embodiment>

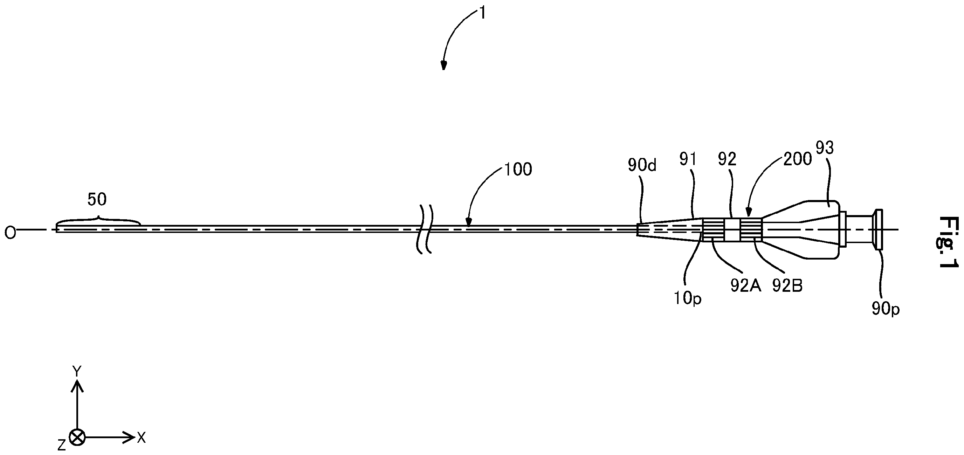

Fig. 1 is an explanatory diagram illustrating a configuration of a catheter 1 of a first embodiment. The catheter 1 is inserted into a living lumen such as a vascular system, a lymph gland system, a biliary system, a urinary tract system, a respiratory system, a digestive system, a secretory gland, and a reproductive organ to be used to diagnose or treat the inside of the living lumen. The catheter 1 includes a tube-shaped (tubular) main body portion 100 and a connector 200 connected to a proximal end side of the main body portion 100. A distal end flexible portion 50 configured to be curvable is provided on a distal end side of the catheter 1.

In Fig. 1, an axis passing through a center of the catheter 1 is represented by an axial line O (dash-dot-dash line). In an example of Fig. 1, the axial line O coincides with an axis passing through a center of each of the main body portion 100 and the connector 200. However, it may not be required that the axial line O coincides with each central axial line of the main body portion 100 and the connector 200. Fig. 1 illustrates an X-axis, a Y-axis, and a Z-axis orthogonal to one another. The X-axis corresponds to a longitudinal direction (length direction) of the catheter 1, the Y-axis corresponds to a width direction of the catheter 1, and the Z-axis corresponds to a height direction of the catheter 1. The left side (- X-axial direction) in Fig. 1 is referred to as "distal end side" of the catheter 1 and each constituent component, and the right side in Fig. 1 (+ X-axial direction) is referred to as "proximal end side" of the catheter 1 and each constituent component. As for the catheter 1 and each constituent component, an end located on the distal end side is referred to as a "distal end", and the distal end and the vicinity thereof are referred to as a "distal end portion". Further, an end located on the proximal end side is referred to as a "proximal end", and the proximal end and the vicinity thereof are referred to as a "proximal end portion". The distal end side is inserted inside a living body, and the proximal end side is operated by an operator such as a doctor. The same is true in the drawings from Fig. 1 onward.

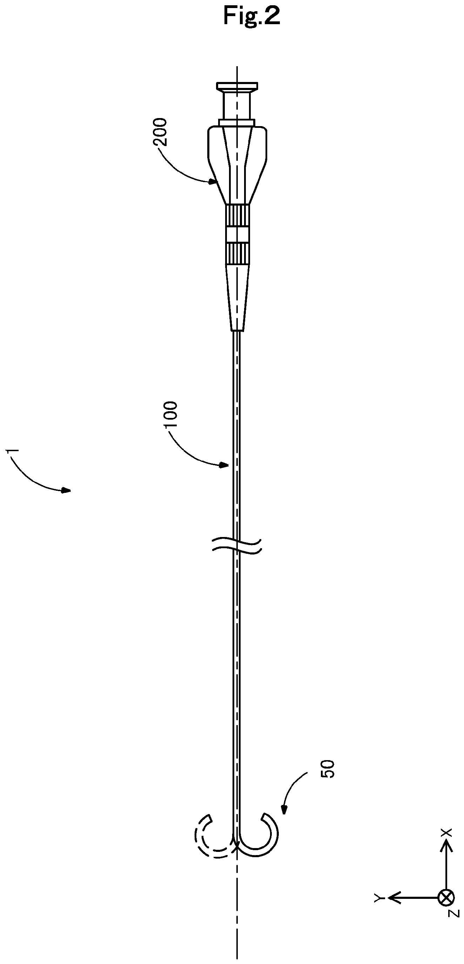

Fig. 2 is an explanatory diagram illustrating the catheter 1 with the distal end flexible portion 50 being curved. As will be described in detail later, in the catheter 1 of the present embodiment, the distal end flexible portion 50 can be curved as illustrated in Fig. 2 by operating an operating wire (described later).

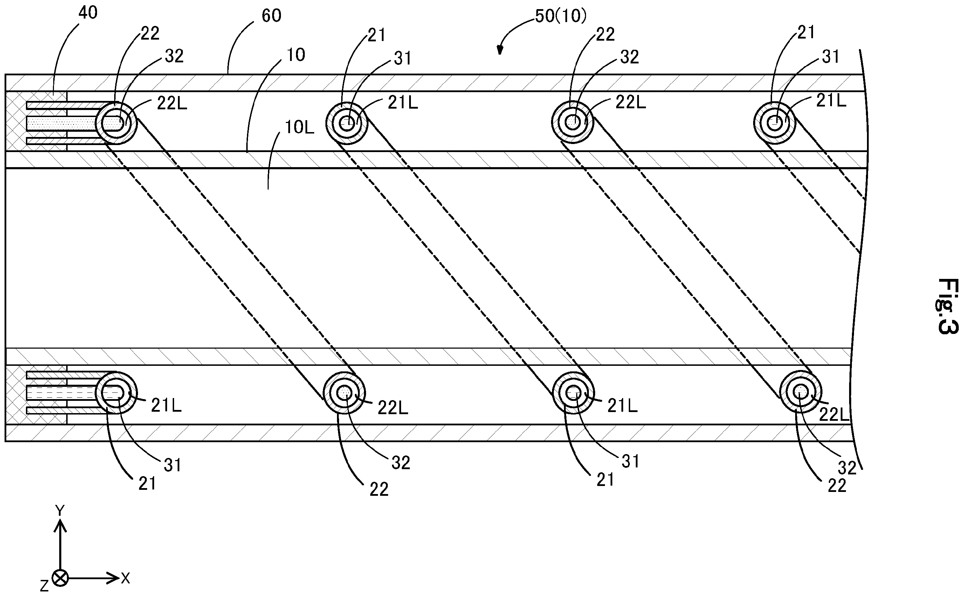

Fig. 3 is an explanatory diagram illustrating a longitudinal sectional configuration of a part of the distal end flexible portion 50 of the catheter 1. As illustrated in Fig. 3, the main body portion 100 includes an outer tube 60, a main tube 10, a first sub tube 21, a second sub tube 22, a first operating wire 31, a second operating wire 32, and a holding portion 40. The outer tube 60 is an elongated member extending along the longitudinal direction (axial line O) of the catheter 1 and is a hollow, substantially cylindrical member. An outer diameter and a length of the outer tube 60 can be freely determined.

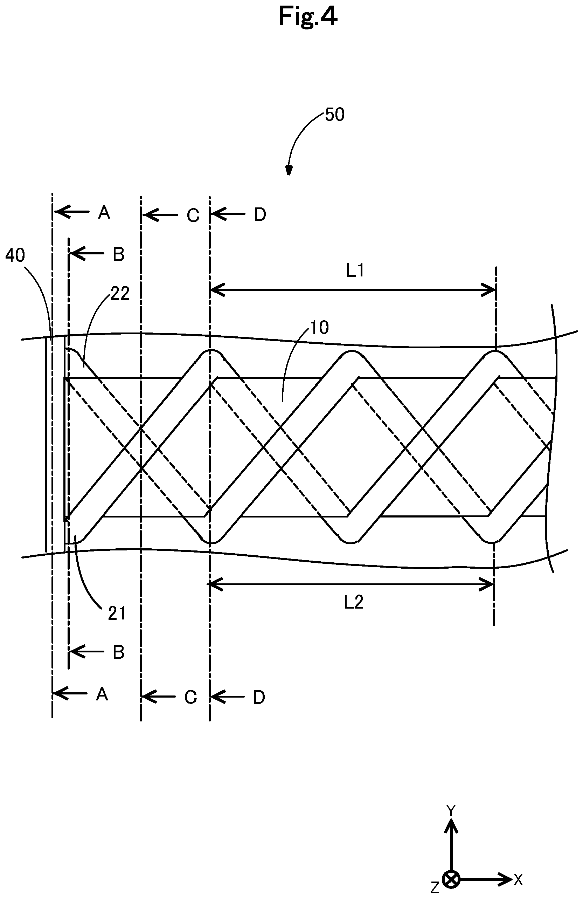

Similarly to the outer tube 60, the main tube 10 is an elongated member extending along the longitudinal direction (axial line O) of the catheter 1 and is a hollow, substantially cylindrical member. That is, the main tube 10 configures an inner cavity (a main lumen 10L) extending across a distal end of the main tube 10 and a proximal end thereof. Fig. 4 is an explanatory diagram of a configuration of the distal end flexible portion 50. As illustrated In Fig. 4, the main tube 10 is arranged in the outer tube 60. An outer diameter of the main tube 10 is smaller than an inner diameter of the outer tube 60. A length of the main tube 10 is substantially the same as a length of the outer tube 60.

The first sub tube 21 is a hollow, elongated member having a substantially cylindrical shape. That is, the first sub tube 21 configures an inner cavity (a first sub lumen 21L) extending across a distal end of the first sub tube 21 and a proximal end thereof. Similarly to the first sub tube 21, the second sub tube 22 is a hollow, elongated member having a substantially cylindrical shape. That is, the second sub tube 22 configures an inner cavity (a second sub lumen 22L) extending across a distal end of the second sub tube 22 and a proximal end thereof. The first sub tube 21 and the second sub tube 22 are arranged inside the outer tube 60 and outside the main tube 10.

As illustrated in Fig. 4, the first sub tube 21 and the second sub tube 22 are arranged around the main tube 10 and are spirally formed around the main tube 10. In other words, the first sub lumen 21L and the second sub lumen 22L are spirally formed around the main lumen 10L.

In the present embodiment, a spiral pitch of the first sub tube 21 (a length L1 illustrated in Fig. 4) and a spiral pitch of the second sub tube 22 (a length L2 illustrated in Fig. 4) are substantially the same, and can be appropriately set within a range of 15 mm or more and 150 mm or less, for example.

The first sub tube 21 and the second sub tube 22 are each wound by a half turn or more (for example, four times) in the distal end flexible portion 50. In other words, the first sub lumen 21L and the second sub lumen 22L are each wound by a half turn or more in the distal end flexible portion 50.

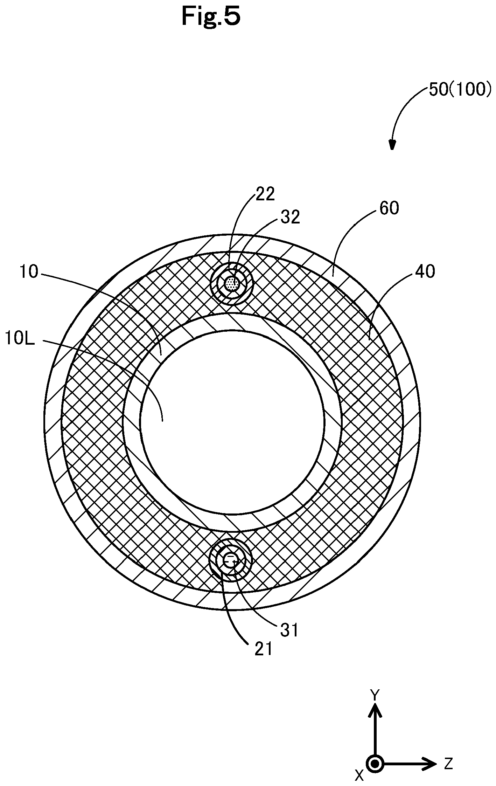

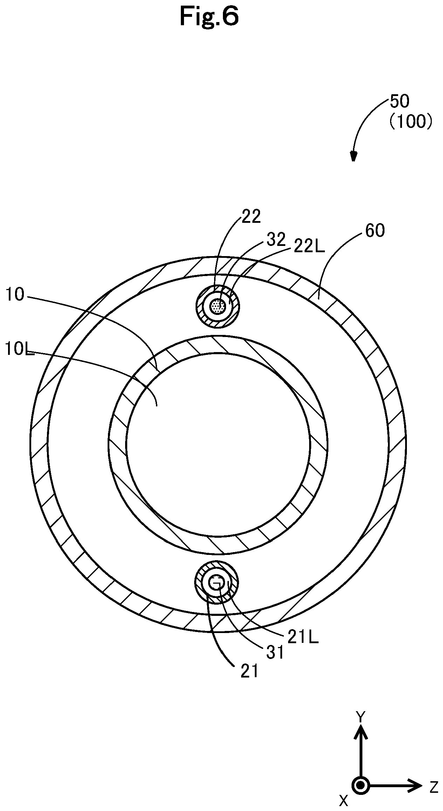

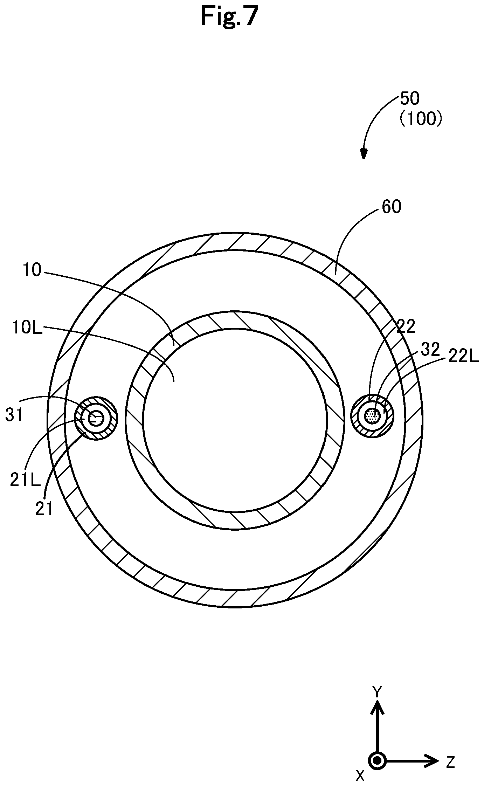

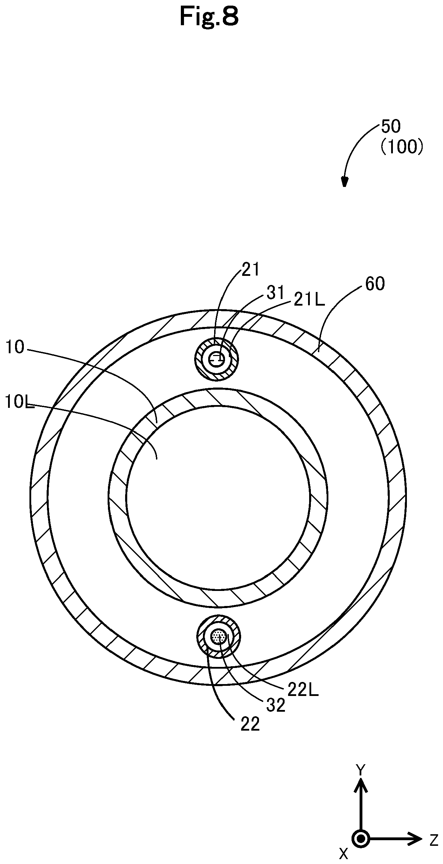

Fig. 5 is a cross-sectional view taken along A-A in Fig. 4, Fig. 6 is a cross-sectional view taken along B-B, Fig. 7 is a cross-sectional view taken along C-C, and FIG. 8 is a cross-sectional view taken along D-D. As illustrated in Figs. 5 to 8, the first sub tube 21 and the second sub tube 22 are each arranged at substantially equal intervals in a circumferential direction of the main tube 10. In other words, the first sub lumen 21L and the second sub lumen 22L are each arranged at substantially equal intervals in the circumferential direction of the main lumen 10L. Here, the "substantially equal intervals" include a tolerance of about ± 20°, for example. The first sub tube 21 and the second sub tube 22 are each spirally wound around the main tube 10, with the main tube 10 as a center, while maintaining a relative positional relationship with each other.

As illustrated in Figs. 3 and 5 to 8, the first operating wire 31 is a solid and elongated member. The first operating wire 31 is arranged in the first sub tube 21, that is, in the first sub lumen 21L, and is spirally wound, similarly to the first sub lumen 21L of the first sub tube 21. As illustrated in Figs. 3 and 5, the distal end portion of the first operating wire 31 is integrally held by the holding portion 40 with the outer tube 60, the main tube 10, and the first sub tube 21. The proximal end portion of the first operating wire 31 is wound around and fixed by a first winding portion 92A described later (Fig. 1).

As illustrated in Figs. 3 and 5 to 8, the second operating wire 32 is a solid and elongated member, similarly to the first operating wire 31. The second operating wire 32 is arranged in the second sub tube 22, that is, in the second sub lumen 22L, and is spirally wound, similarly to the second sub lumen 22L of the second sub tube 22. As illustrated in Figs. 3 and 5, the distal end portion of the second operating wire 32 is integrally held by the holding portion 40 with the outer tube 60, the main tube 10, and the second sub tube 22. The proximal end portion of the second operating wire 32 is wound around and fixed by a second winding portion 92B described later (Fig. 1). In the following description, if there is no need of distinguishing the first operating wire 31 and the second operating wire 32, these are simply referred to as "operating wire".

The holding portion 40 is arranged at the distal end portion of the outer tube 60, and is a member that integrally holds a distal end side of the main tube 10, a distal end side of the first operating wire 31, a distal end side of the second operating wire 32, a distal end side of the first sub tube 21, and a distal end side of the second sub tube 22. As illustrated in Figs. 3 and 5, the holding portion 40 is provided inside the outer tube 60 and over the entire outside of the main tube 10, but is not provided inside the main tube 10. Thus, it is possible to use the main lumen 10L of the main tube 10 as a guide wire lumen for inserting a guide wire into the catheter 1 or as a lumen for a chemical liquid for supplying a chemical liquid to a living tissue.

As illustrated in Fig. 1, the connector 200 is a member arranged at a proximal end portion 10p of the main body portion 100 and gripped by an operator. The connector 200 includes a connector main body portion 91, a winding portion 92, and a blade portion 93. The connector main body portion 91 has a hollow shape including an opening at each of a distal end portion 90d and a proximal end portion 90p and being formed therein with an inner cavity communicating each opening. A distal end side of the connector main body portion 91 is joined with a part of the proximal end side of the main body portion 100 being inserted therein. For joining, any adhesive such as an epoxy-based adhesive can be utilized.

The winding portion 92 includes the first winding portion 92A and the second winding portion 92B. The first winding portion 92A and the second winding portion 92B each include a tubular or rod-shaped wire wound portion and a grip portion provided on the outside of the wire wound portion. The wire wound portion of the first winding portion 92A is wound and fixed with the proximal end portion of the first operating wire 31. When the grip portion of the first winding portion 92A is rotated clockwise, the proximal end portion of the first operating wire 31 is wound around the wire wound portion, and as a result, it is possible to perform an operation of pulling the proximal end side of the first operating wire 31. When the grip portion of the first winding portion 92A is rotated counterclockwise, the proximal end portion of the first operating wire 31 is fed out from the wire wound portion, and as a result, it is possible to perform an operation of loosening the first operating wire 31. Similarly, the wire wound portion of the second winding portion 92B is wound and fixed with the proximal end portion of the second operating wire 32. When the grip portion of the second winding portion 92B is rotated clockwise, it is possible to perform an operation of pulling the proximal end side of the second operating wire 32. When the grip portion of the second winding portion 92B is rotated counterclockwise, it is possible to perform an operation of loosening the second operating wire 32.

The blade portion 93 is a blade-shaped member having two blades provided on the proximal end side of the connector main body portion 91. The blade portion 93 is used by the operator to grip the catheter 1. The blade portion 93 may have any shape and may be omitted. The blade portion 93 may be joined to the connector main body portion 91, or may be integrally formed with the connector main body portion 91.

The outer tube 60, the main tube 10, the first sub tube 21, and the second sub tube 22 preferably have antithrombotic, flexible, and biocompatible properties, and may be formed of a resin material or a metal material. Examples of the resin material may include a polyamide resin, a polyolefin resin, a polyester resin, a polyurethane resin, a silicone resin, and a fluororesin. Examples of the metal material may include stainless steel such as SUS304, a nickel-titanium alloy, and a cobalt-chromium alloy. In addition, if gold, platinum, and tungsten, which are radiopaque materials, or an alloy containing these elements are adopted, it is possible to improve visibility under X-ray fluoroscopy, hence preferable.

The first operating wire 31 and the second operating wire 32 may be formed of, for example, a metal material such as stainless steel including SUS304, a nickel titanium alloy, a cobalt chrome alloy, and a tungsten alloy. The holding portion 40 is preferably flexible and may be formed of, for example, a resin material such as polyurethane and polyurethane elastomer. The connector 200 may be formed of, for example, a resin material such as polyamide, polypropylene, polycarbonate, polyacetal, and polyether sulfone. It is noted that each of the above-mentioned materials is merely an example, and each member may be formed by using a known material other than the materials mentioned above.

In the main body portion 100 of the catheter 1, the distal end flexible portion 50 is more flexible than the other parts of the main body portion 100. Various methods may be used to modify the flexibility of the main body portion 100. For example, thicknesses of the outer tube 60, the main tube 10, the first sub tube 21, and the second sub tube 22 may be reduced in the distal end flexible portion 50. Parts of the outer tube 60, the main tube 10, the first sub tube 21, and the second sub tube 22, corresponding to the distal end flexible portion 50, may be formed of a material having a lower rigidity (a material having a higher flexibility) than the other parts. The distal end flexible portion 50 may be provided at a site of a portion between the distal end of the main body portion 100 and a position separated by a predetermined distance. For example, the predetermined distance may be 100 mm, or a part of the predetermined distance, for example, the distal end flexible portion 50 may be formed in a range of 20 to 50 mm from the distal end.

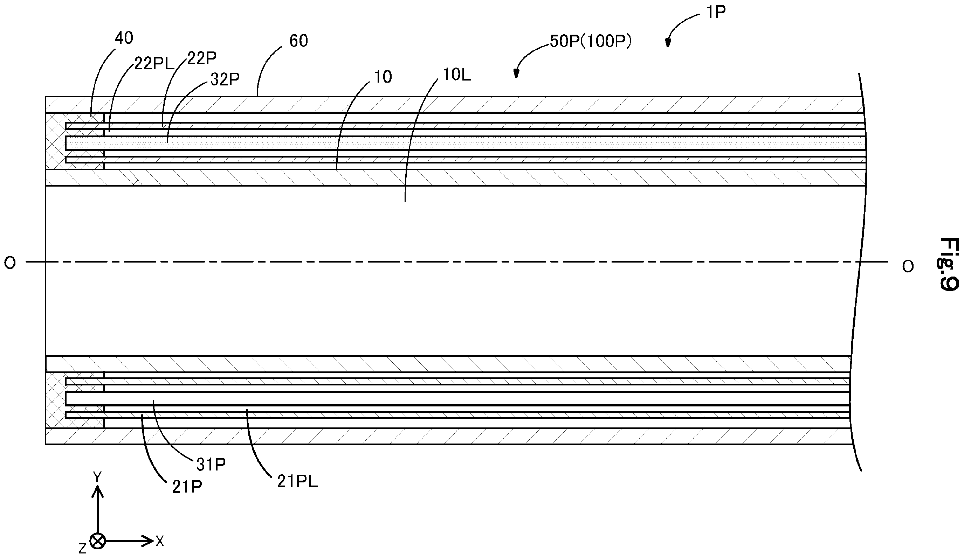

Prior to the description of an effect of the catheter 1 of the present embodiment (first embodiment), a catheter of the prior art will be described. Fig. 9 is an explanatory diagram for describing a configuration of a catheter 1P of the prior art. Fig. 9 is a longitudinal cross section corresponding to Fig. 3. In the catheter 1P of the prior art, a first sub tube 21P and a second sub tube 22P are formed linearly along the axial line O in a distal end flexible portion 50P. Therefore, in the catheter 1P of the prior art, a first operating wire 31P and a second operating wire 32P are also formed linearly.

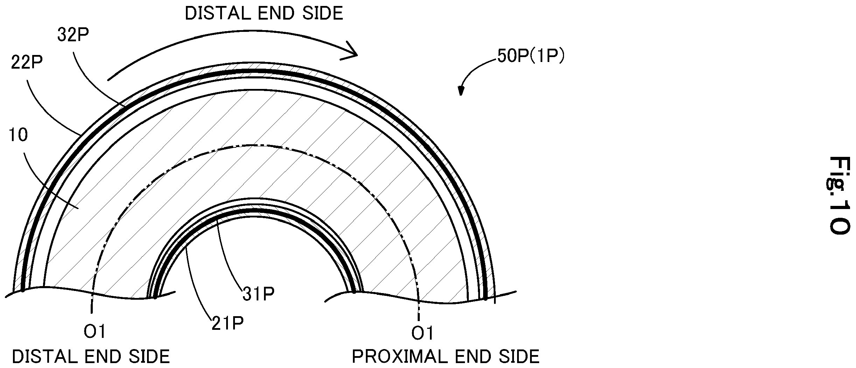

Fig. 10 is an explanatory diagram illustrating a case where the distal end flexible portion 50P of the catheter 1P of the prior art is curved. In Fig. 10, to distinguish the main tube 10, the first sub tube 21P, and the second sub tube 22P, a different hatching is added. When the first operating wire 31P is pulled to curve the distal end flexible portion 50P, tension is generated in the second sub tube 22P and the second operating wire 32P, and thus, a drag force against the tension is generated (arrow in Fig. 10) in the second sub tube 22P and the second operation wire 32P. Thus, due to the drag force generated in the second sub tube 22P and the second operating wire 32P when the first operating wire 31P is pulled to further curve the distal end flexible portion 50P, it is difficult to pull the first operating wire 31P. That is, it is difficult to curve the distal end flexible portion 50P.

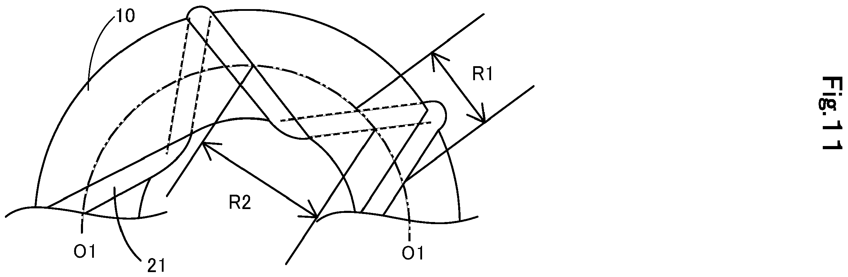

Fig. 11 is an explanatory diagram of an effect of the catheter 1 of the present embodiment. Fig. 11 illustrates the main tube 10 and the first sub tube 21 in a state where the distal end flexible portion 50 of the catheter 1 is curved. For example, if the distal end flexible portion 50 is curved by pulling the first operating wire 31 arranged in the first sub lumen 21L inside the first sub tube 21, the first sub tube 21 is deformed as the main tube 10 is curved. In Fig. 11, beyond a central axial line O1 of the main tube 10, outside the curving of the main tube 10 (in Fig. 11, an example of such a range is allotted with reference symbol Rl), the main tube 10 is stretched and tension is exerted on the first sub tube 21. On the other hand, beyond the central axial line O1 of the main tube 10, inside the curving of the main tube 10 (in Fig. 11, an example of such a range is allotted with reference symbol R2), the main tube 10 contracts and the tension on the first sub tube 21 is loosened. That is, if the first sub tube 21 is wound by a half turn to include the part where the tension is generated and the part where the tension is loosened, the tensions are canceled out in such parts. In the catheter 1 of the present embodiment, the first sub tube 21 is spirally wound by a half turn or more in the distal end flexible portion 50, and thus, the tension generated in the first sub tube 21 is canceled out. As a result, a force generated in the first operating wire 31 arranged in the first sub lumen 21L of the first sub tube 21 is suppressed. Therefore, it is possible to improve the operability when the operating wire is operated in a state where the distal end flexible portion 50 of the catheter 1 is curved.

According to the catheter 1 of the present embodiment, the second sub tube 22 is spirally formed, similarly to the first sub tube 21. Therefore, it is possible to obtain a similar effect when the second operating wire 32 arranged in the second sub lumen 22L of the second sub tube 22 is pulled to curve the distal end flexible portion 50.

In the catheter 1 of the present embodiment, the first sub tube 21 and the second sub tube 22 are arranged substantially evenly in the circumferential direction of the main tube 10. Therefore, it is possible to easily curve the distal end flexible portion 50 in a direction desired by the operator.

In the catheter 1 of the present embodiment, the main lumen 10L is configured by the main tube 10, the first sub lumen 21L is configured by the first sub tube 21, and the second sub lumen 22L is configured by the second sub tube 22. Therefore, it is possible to easily manufacture the catheter 1 spirally formed with the first sub lumen 21L and the second sub lumen 22L.

### <Second Embodiment>

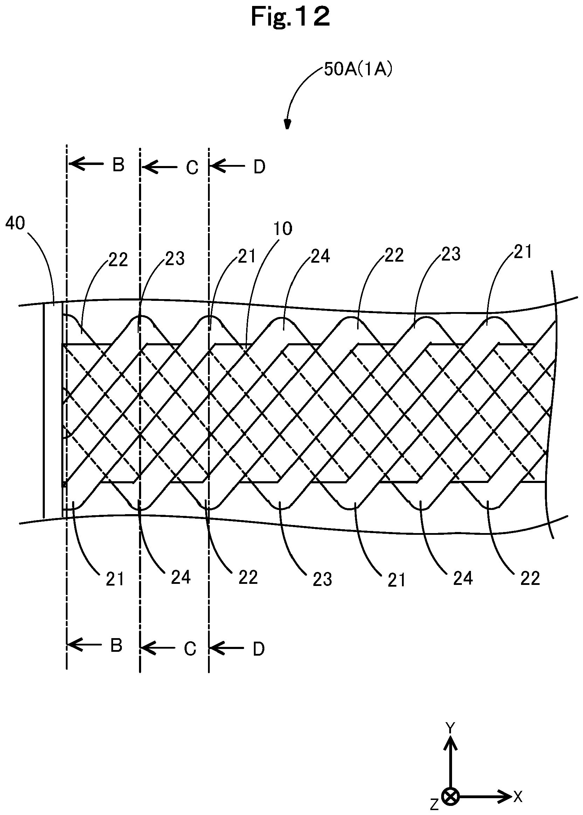

Fig. 12 is an explanatory diagram of a configuration of a distal end flexible portion 50A of a catheter 1A of a second embodiment. In addition to the configuration of the catheter 1 of the first embodiment, the catheter 1A of the second embodiment further includes a third sub tube 23, a third operating wire 33 arranged in the third sub tube 23, a fourth sub tube 24, and a fourth operating wire 34 arranged in the fourth sub tube 24. In the following description, if there is no need for distinguishing the first to fourth sub tubes, these are also simply referred to as "sub tube", and if there is no need for distinguishing the first to fourth operating wires, these are also simply referred to as "operating wire".

As illustrated in Fig. 12, similarly to the first sub tube 21 and the second sub tube 22, the third sub tube 23 and the fourth sub tube 24 are hollow, elongated members having a substantially cylindrical shape, arranged around the main tube 10, and spirally formed around the main tube 10.

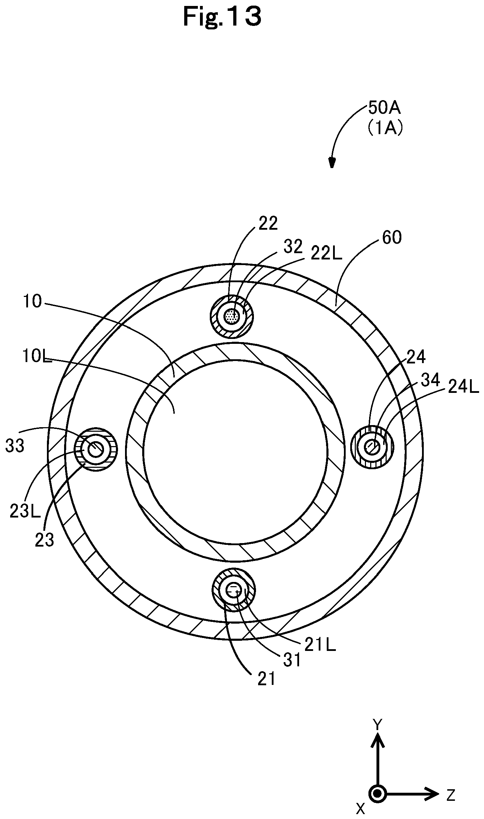

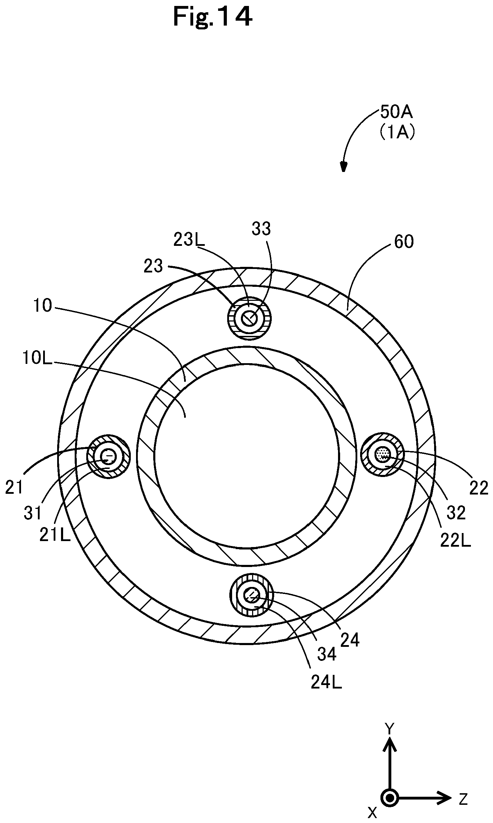

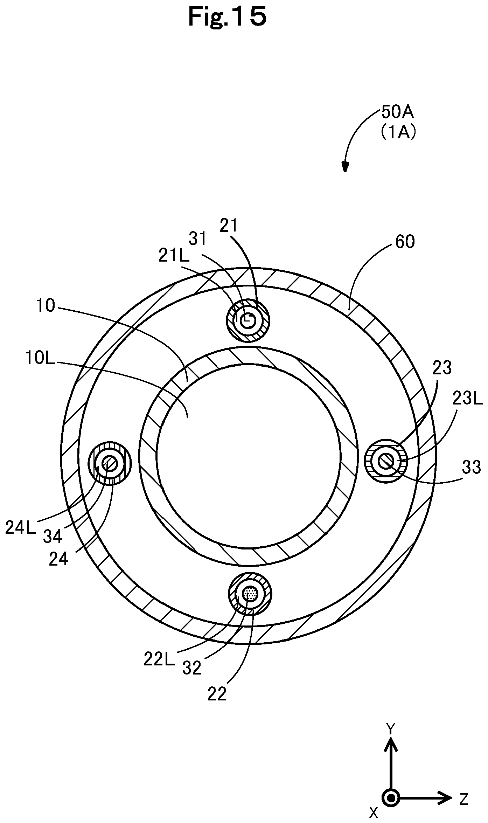

Fig. 13 illustrates a cross section taken along B-B in Fig. 12, Fig. 14 illustrates a cross section taken along C-C, and Fig. 15 illustrates a cross section taken along D-D. A cross-sectional position in Fig. 12 coincides with a cross-sectional position in Fig. 4. As illustrated, the third sub tube 23 configures a third sub lumen 23L, and the third operating wire 33 is arranged in the third sub lumen 23L. Similarly, the fourth sub tube 24 configures a fourth sub lumen 24L, and the fourth operating wire 34 is arranged in the fourth sub lumen 24L.

The first sub tube 21, the second sub tube 22, the third sub tube 23, and the fourth sub tube 24 are each arranged at substantially equal intervals in the circumferential direction of the main tube 10. In other words, the first sub lumen 21L, the second sub lumen 22L, the third sub lumen 23L, and the fourth sub lumen 24L are each arranged at substantially equal intervals in the circumferential direction of the main lumen 10L. The first sub tube 21, the second sub tube 22, the third sub tube 23, and the fourth sub tube 24 are each spirally wound around the main tube 10, with the main tube 10 as a center, while maintaining relative positional relationships with one another.

The first sub tube 21, the second sub tube 22, the third sub tube 23, and the fourth sub tube 24 are each wound by a half turn or more (for example, four times) in the distal end flexible portion 50. In other words, the first sub lumen 21L, the second sub lumen 22L, the third sub lumen 23L, and the fourth sub lumen 24L are each wound by a half turn or more in the distal end flexible portion 50.

Even in such a configuration, each of the sub tubes is spirally wound by a half turn or more, and thus, the tensions generated in the sub tubes are canceled out, and the force generated in the operating wire can be suppressed. The four operating wires are provided, and thus, it is possible to more accurately operate the distal end flexible portion 50A in the curving direction, than the catheter 1 of the first embodiment.

### <Modifications of Embodiment>

The disclosed embodiments are not limited to the above-described embodiments, and may be implemented in various modes without departing from the spirit of the disclosed embodiments. The following modifications can be applied, for example.

- The number of sub lumens is not limited to the number in the above-described embodiments. For example, three or five or more sub lumens may be provided.

- In the above-described embodiments, an example in which the sub lumens are arranged at substantially equal intervals in the circumferential direction of the main lumen is described, but it is not required that the sub lumens are arranged at substantially equal intervals.

- In the above-described embodiments, an example of a catheter obtained by using a hollow tube having a substantially circular tubular shape to form the inner cavity (lumen) is described, but the catheter may be formed by using a multi-lumen tube including the main lumen and the sub lumen in the above-described embodiments.
- In the above-described embodiments, a blade layer formed by knitting wires may be further provided. If the blade layer is provided, the flexibility of the main body portion 100 may be changed depending on the presence or absence of the blade layer and the sparseness or density of the blade layer, for example. Specifically, in the main body portion 100, the distal end flexible portion 50 may not include the blade layer, and another part may include the blade layer. In the main body portion 100, the distal end flexible portion 50 may have a lower density of the blade layer than other parts.
- It may suffice that the sub lumen is spirally formed by a half turn or more in at least the distal end flexible portion 50. For example, in a part other than the distal end flexible portion 50 in the main body portion 100, the sub lumen may be formed linearly, and may be formed spirally with a spiral pitch larger than that of the distal end flexible portion 50. In a part other than the distal end flexible portion 50 of the main body portion 100, the sub lumen may be formed spirally with the same pitch as the spiral pitch in the distal end flexible portion 50.

Although the aspects have been described based on the embodiments and the modifications, the embodiments of the above-described aspects are for facilitating understanding of the aspects, and do not limit the aspects. The aspects can be modified and improved without departing from the spirit of the aspects and the scope of the claims, and equivalent aspects are included in the aspects. Further, unless a technical feature is described as essential in the present specification, the technical feature may be omitted as appropriate.

### DESCRIPTION OF REFERENCE NUMERALS

1, 1A, 1P ... Catheter
10 ... Main tube
10L ... Main lumen
10p ... Proximal end portion
21, 21P ... First sub tube
21L ... First sub lumen
22, 22P ... Second sub tube
22L ... Second sub lumen
23 ... Third sub tube
23L ... Third sub lumen
24 ... Fourth sub tube
24L ... Fourth sub lumen
31, 31P ... First operating wire
32, 32P ... Second operating wire
33 ... Third operating wire
34 ... Fourth operating wire
40 ... Holding portion
50, 50A, 50P ... Distal end flexible portion
60 ... Outer tube
90d ... Distal end portion
90p ... Proximal end portion
91 ... Connector main body portion
92 ... Winding portion
92A ... First winding portion
92B ... Second winding portion
93 ... Blade portion
100... Main body portion
200 ... Connector
O ... Axial line
O1 ... Central axial line

## Claims

1. A catheter, comprising:
a main lumen;
a plurality of sub lumens arranged around the main lumen and spirally formed around the main lumen;
a plurality of operating wires arranged in each of the plurality of sub lumens;
a distal end flexible portion arranged on a distal end side of the catheter and curvable by the plurality of operating wires; and
a holding portion arranged at a distal end of the distal end flexible portion and holding each of the plurality of operating wires, wherein
each of the plurality of sub lumens is wound by a half turn or more in the distal end flexible portion.

2. The catheter according to claim 1, wherein
the plurality of sub lumens are each arranged at substantially equal intervals in a circumferential direction of the main lumen.

3. The catheter according to any one of claims 1 and 2, further comprising:
a hollow tubular main tube configuring the main lumen;
a plurality of hollow tubular sub tubes configuring each of the plurality of sub lumens; and
a hollow tubular outer tube containing the main tube and the sub tubes.

4. The catheter according to any one of claims 1 to 3, wherein
the plurality of sub lumens include a first sub lumen and a second sub lumen, and
the plurality of operating wires include a first operating wire arranged in the first sub lumen and a second operating wire arranged in the second sub lumen.
